# EUROPÄISCHE PATENTANMELDUNG

(11) **EP 0 806 189 A1**
(43) Veröffentlichungstag der Anmeldung: **12.11.1997**
(21) Anmeldenummer: 97107629.4
(22) Anmeldetag: 09.05.1997
(51) Int. Cl.: A61F 2/04, A61M 25/00

(54) **Vorrichtung zur Drainage mit einem Drainagerohr**

(30) Priorität: 10.05.1996 DE 19618966
(71) Anmelder: Variomed AG, 9496 Balzers (LI)
(72) Erfinder:
(74) Vertreter: Pellkofer, Dieter Dr.

(57) **Zusammenfassung**

Es wird eine Vorrichtung zur Drainage an einem Patienten mit einem Drainagerohr, einem mit dem Drainagerohr koppelbaren Führungsrohr und einem Schiebeelement beschrieben. Zumindest ein distaler Endabschnitt des Drainagerohrs ist innerhalb des Führungsrohrs angeordnet. Das Schiebeelement ist innerhalb des Führungsrohrs zum Entkoppeln von Drainagerohr und Führungsrohr gegen den distalen Endabschnitt des Drainagerohrs verschiebbar.

## Beschreibung

Die vorliegende Erfindung betrifft eine Vorrichtung zur Drainage an einem Patienten mit einem Drainagerohr, einem mit dem Drainagerohr koppelbaren Führungsrohr und einem Schiebeelement.

Drainagerohre werden im Harnleiter zwischen der Niere und der Blase eingesetzt, um einen guten Urindurchfluß von der Niere zur Blase zu gewährleisten. Um eine Verankerung im Bereich zwischen der Niere und der Blase zu ermöglichen, sind Drainagerohre üblicherweise federelastisch ausgebildet, wobei an einem oder an beiden Enden eine J-artige Krümmung oder eine Krümmung in Form eines sog. "Schweineschwanzes" vorgesehen ist. Zum Einführen des Drainagerohres in die Harnröhre wird das Drainagerohr auf das proximale Ende des Führungsrohrs aufgeschoben, bis es über seine gesamte Länge an der Außenseite des Führungsrohres anliegt, so daß das Drainagerohr eine langestreckte Form annimmt. Anschließend wird über das distale Ende des Führungsrohrs ein Schieberohr auf das Führungsrohr so weit aufgeschoben, bis das proximale Ende des Schieberohres an dem distalen Ende des Drainagerohres zur Anlage kommt.

Die aus Drainagerohr, Führungsrohr und Schiebeelement bestehende Vorrichtung wird in den Harnleiter eingeführt, bis das proximale Ende des Drainagerohrs in der Niere angeordnet ist. Durch Zurückziehen des Führungsrohres unter gleichzeitigem Festhalten des Schieberohrs kann das Drainagerohr von dem Führungsrohr abgestreift werden, so daß es im Harnleiter zwischen der Niere und der Blase plaziert ist und das Führungsrohr zusammen mit dem Schieberohr aus dem Harnleiter herausgezogen werden kann.

Damit ein versehentliches Herabrutschen des Drainagerohrs vom den Führungsrohr während des Positionierens verhindert wird, muß das Drainagerohr relativ straff auf dem Führungsrohr aufgesetzt bzw. über separate Fixierelemente mit diesem fixiert sein. Das Schieberohr muß sich hingegen relativ leichtgängig auf dem Führungselement verschieben lassen, damit das Abstreifen des Drainagerohrs vom Führungsrohr nicht durch zusätzliche, zwischen dem Schieberohr und dem Führungsrohr wirkende Reibungskräfte erschwert wird.

Die leichtgängige Verschiebbarkeit des Schieberohrs auf dem Führungsrohr kann dazu führen, daß beim Einschieben der Vorrichtung in den Harnleiter das außen liegende Schieberohr aufgrund der Reibung zwischen der Außenwand des Schieberohrs und der Innenwand des Harnleiters gegenüber dem Drainagerohr verschoben wird, so daß zwischen Drainagerohr und Schieberohr ein Spalt ausgebildet ist. Wenn zum Abstreifen des Drainagerohrs vom Führungsrohr das Schieberohr in Richtung zum Drainagerohr hin verschoben wird, können Teile der Harnleiterinnenwand in dem zwischen dem proximalen Ende des Schieberohrs und dem distalen Ende des Drainagerohrs vorhandenen Spalt eingeklemmt werden, was zu Verletzungen führen kann.

Der vorliegenden Erfindung liegt die Aufgabe zugrunde, eine Vorrichtung der eingangs genannten Art so auszubilden, daß eine sichere und einfache Einführung des Drainagerohres möglich ist, wobei gleichzeitig ein einfacher Aufbau und eine kostengünstige Herstellung der Vorrichtung erreicht werden soll.

Diese Aufgabe wird ausgehend von einer Vorrichtung der eingangs genannten Art gemäß der Erfindung dadurch gelöst, daß zumindest ein distaler Endabschnitt des Drainagerohrs innerhalb des Führungsrohrs angeordnet ist und daß das Schiebeelement innerhalb des Führungsrohrs zum Entkoppeln von Drainagerohr und Führungsrohr gegen den distalen Endabschnitt des Drainagerohrs verschiebbar ist.

Durch die Anordnung sowohl eines distalen Endabschnittes des Drainagerohrs als auch des Schiebeelements innerhalb des Führungsrohrs ist gewährleistet, daß ein durch die leichtgängige Verschiebbarkeit des Schiebeelements gegenüber dem Führungsrohr entstehender Spalt innerhalb des Führungsrohrs angeordnet ist und somit nicht zu einem Einklemmen der Harnleiterwand führen kann.

Es ist sogar möglich, das Drainagerohr lediglich zusammen mit dem Führungsrohr einzuführen und erst nach Erreichen der Endposition des Drainagerohrs das Schiebeelement außerhalb des Körpers des Patienten in das proximale Ende des Führungsrohrs einzusetzen und bis zum distalen Ende des Drainagerohres zu schieben. Da das Schiebeelement ausschließlich innerhalb des Führungsrohres geführt wird, werden die Harnleiterinnenwand durch das nachträgliche Einführen des Schiebelements in keiner Weise berührt.

Nach einer weiteren Ausführungsform der Erfindung sind das Drainagerohr und das Führungsrohr über den distalen Endabschnitt des Drainagerohrs miteinander insbesondere kraftschlüssig verkoppelbar. Dies kann insbesondere dadurch erreicht werden, daß der Außendurchmesser des distalen Endabschnitts des Drainagerohrs im wesentlichen gleich dem Innendurchmesser des Führungsrohrs ist, so daß der distale Endabschnitt des Drainagerohrs bevorzugt im wesentlichen im Preßsitz innerhalb des Führungsrohr angeordnet ist. Auf diese Weise wird durch die zwischen der Außenseite des distalen Endabschnitts und der Innenseite des Führungsrohrs wirkende Reibungskraft eine im wesentlichen kraftschlüssige Kopplung gebildet.

Durch die kraftschlüssige Kopplung ist gewährleistet, daß das Drainagerohr nicht versehentlich während des Positionierens von dem Führungsrohr abrutschen kann, wobei gleichzeitig eine Verdrehung des Drainagerohrs durch eine entsprechende Verdrehung des Führungsrohrs erreichbar ist. Eine solche Verdrehung des Drainagerohres ist zum einen notwendig, um eine Durchführung des gebogenen Drainagerohres durch Kurven des Harnleiters zu erleichtern und zum anderen um eine optimale Positionierung des gebogenen Drainagerohres in seiner Endposition zwischen Niere und Blase zu erreichen.

Nach einer weiteren vorteilhaften Ausführungsform der Erfindung ist der distale Endabschnitt des Drainagerohrs als insbesondere röhrenförmiges, separates Element ausgebildet, das mit dem Hauptabschnitt des Drainagerohrs verbunden, insbesondere verklebt ist. Durch die separate Ausbildung ist eine besonders einfache Fertigung des erfindungsgemäß ausgebildeten Drainagerohres möglich, da sowohl der Hauptabschnitt des Drainagerohres als auch der distale Endabschnitt als rohrförmige Elemente ausgebildet sein können, welche ineinandergeschoben und miteinander verbunden werden. Außer durch eine Verklebung kann die Verbindung auch auf jede weitere geeignete Weise wie beispielsweise eine Verschmelzung durchgeführt werden. Grundsätzlich können der Hauptabschnitt und der distale Endabschnitt des Drainagerohrs auch einstückig beispielsweise als Gußstück ausgebildet sein.

Nach einer weiteren bevorzugten Ausführungsform der Erfindung besitzen der Hauptabschnitt des Drainagerohrs und das Führungsrohr im wesentlichen denselben Außendurchmesser. Auf diese Weise kann eine gleichmäßige Außenfläche der aus Drainagerohr und Führungsrohr zusammengesetzten Vorrichtung erreicht werden, so daß keine den Einführprozeß behindernden Unregelmäßigkeiten, wie z. B. Stufen, an der Außenseite der Vorrichtung ausgebildet sind.

Grundsätzlich kann der Außendurchmesser des Hauptabschnitts des Drainagerohrs auch gleich dem Außendurchmesser des distalen Endabschnitts des Drainagerohrs sein, so daß das Drainagerohr über seine gesamte Ausdehnung einen Außendurchmesser besitzt, der dem Innendurchmesser des Führungsrohres entspricht. Bei dieser Ausbildung kann das Drainagerohr über einen Teil oder über seine gesamte Länge in das Führungsrohr eingeschoben werden, so daß die Außenseite der einzuführenden Vorrichtung ausschließlich durch die Außenseite des Führungsrohres gebildet wird. Dadurch wird erreicht, daß an der Außenseite der einzuführenden Vorrichtung keinerlei Spalte oder sonstige Unregelmäßigkeiten vorhanden sind, so daß ein reibungsloses Einführen der Vorrichtung möglich ist.

Nach einer weiteren vorteilhaften Ausführungsform der Erfindung sind das Drainagerohr und das Führungsrohr über am distalen Ende des Hauptabschnitts des Drainagerohrs und am proximalen Ende des Führungsrohrs vorgesehene Kopplungselemente miteinander koppelbar. Durch diese Kopplungselemente wird erreicht, daß zusätzlich zu der durch die Reibungskraft erzeugten kraftschlüssigen Verbindung eine formschlüssige Verbindung zwischen Drainagerohr und Führungsrohr besteht, so daß beim Verdrehen des Führungsrohres ein sicheres Mitverdrehen des Drainagerohres gewährleistet ist.

Die Kopplung zwischen dem Drainagerohr und dem Führungsrohr kann bevorzugt dadurch gebildet werden, daß die abgeschrägten Enden mit der Längsachse des Drainagerohrs bzw. des Führungsrohrs einen Winkel zwischen 90° und 30°, insbesondere zwischen 90° und 45° einschließen. Durch die bei der zusammengesetzten Vorrichtung aneinanderliegenden abgeschrägten Enden des Drainagerohrs und des Führungsrohres wird eine Drehung des Führungsrohres auf das Drainagerohr übertragen, so daß dieses zusammen mit dem Führungsrohr verdreht wird.

Die die Kopplung ermöglichenden Schrägflächen können beispielsweise durch einfaches schräges Abschneiden der Enden des Drainagerohr bzw. des Führungsrohres erreicht werden, so daß eine besonders einfache Herstellung dieser Ausführungsform möglich ist.

Nach einer weiteren bevorzugten Ausführungsform sind das am Drainagerohr vorgesehene Kopplungselement als insbesondere V-förmige Ausnehmung und das am Führungsrohr vorgesehene Kopplungselement als insbesondere im wesentlichen komplementär dazu ausgebildeter Vorsprung ausgebildet. Bei der zusammengesetzten Vorrichtung greift somit der am Führungsrohr vorgesehene Vorsprung in die am Drainagerohr vorgesehene V-förmige Ausnehmung, so daß eine drehfeste Kopplung zwischen diesen beiden Elementen erreicht wird. Die Ausnehmung und der zugehörige Vorsprung können jedoch auch jede andere geeignete Form, beispielsweise eine Rechteckform oder eine Trapezform besitzen. Weiterhin können Ausnehmung und Vorsprung auch vertauscht angeordnet sein.

Bevorzugt ist das Schiebeelement als Schieberohr ausgebildet und insbesondere dessen Außendurchmesser im wesentlichen gleich oder geringfügig kleiner als der Innendurchmesser des Führungsrohrs. Auf diese Weise kann die gesamte Vorrichtung über einen relativ steifen Führungsdraht, der die flexiblen gekrümmten Enden des Drainagerohrs in der zur Einführung erforderlichen langgestreckten Form hält, in den Harnleiter eingeführt werden, wobei durch den gewählten Außendurchmesser des Schieberohrs ein leichtgängiges Verschieben des Verschieberohrs innerhalb des Führungsrohrs gewährleistet ist.

Vorteilhaft sind das Drainagerohr und/oder das Führungsrohr und/oder das Schiebeelement aus körperverträglichem Kunststoff, beispielsweise aus Polyethylen, Polyamid oder Polyurethanelastomeren hergestellt. Dadurch ist gewährleistet, daß eine gute Verträglichkeit des eingesetzten Drainagerohres erzielt wird.

Weitere vorteilhafte Ausführungsformen der Erfindung sind in den Unteransprüchen angegeben.

Die vorliegende Erfindung wird im folgenden anhand eines Ausführungsbeispiels unter Bezugnahme auf die Zeichnungen näher beschrieben; in diesen zeigen:
- Figur 1: ein Drainagerohr mit beidseitig gekrümmten Enden,
- Figur 2: ein Drainagerohr mit einseitig gekrümmten Ende,
- Figur 3: einen Teilschnitt durch eine erfindungsgemäß ausgebildete Vorrichtung im Verbindungsbereich zwischen Drainagerohr und Führungsrohr,
- Figur 4: einen Teilschnitt einer weiteren Ausführungsform im Verbindungsbereich zwischen Drainagerohr und Führungsrohr und
- Figur 5: eine Seitenansicht eines Führungsrohrs mit eingesetztem Schieberohr.

Das in Figur dargestellte Drainagerohr 1 besitzt an seinem proximalen Ende eine ringförmige flexible Umbiegung 2, die nach dem Einsetzen in der Niere angeordnet ist und ein Herausrutschen des Drainagerohrs aus der Niere verhindert.

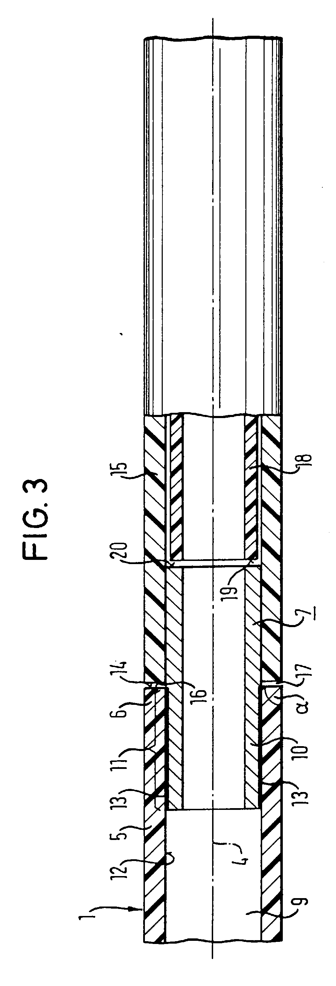

Am distalen Ende des Drainagerohrs 1 ist eine hakenförmige Umbiegung 3 vorgesehen, die sich bezüglich der Längsachse 4 des Drainagerohrs (siehe Figur 3) auf der von der ringförmigen Umbiegung 2 abgewandten Seite des Drainagerohrs 1 erstreckt und eine Fixierung des eingesetzten Drainagerohrs 1 in der Blase gewährleistet.

Das Drainagerohr 1 umfaßt einen Hauptabschnitt 5 mit im wesentlichen konstantem Außendurchmesser, an dessen distales Ende 6 sich ein distaler Endabschnitt 7 des Drainagerohrs 1 anschließt, der einen gegenüber dem Hauptabschnitt 5 verkleinerten Außendurchmesser besitzt.

In der Wand des Hauptabschnitts 5 des Drainagerohrs 1 sind Öffnungen 8 ausgebildet, die den im Inneren des Drainagerohrs 1 ausgebildeten Hohlraum 9 (Fig. 3) mit der außerhalb des Drainagerohrs 1 gelegenen Umgebung verbinden, so daß bei im Harnleiter eingesetztem Drainagerohr 1 durch den Hohlraum 9 und die Öffnungen 8 ein durchgängiger Urinfluß von der Niere zur Blase gewährleistet ist.

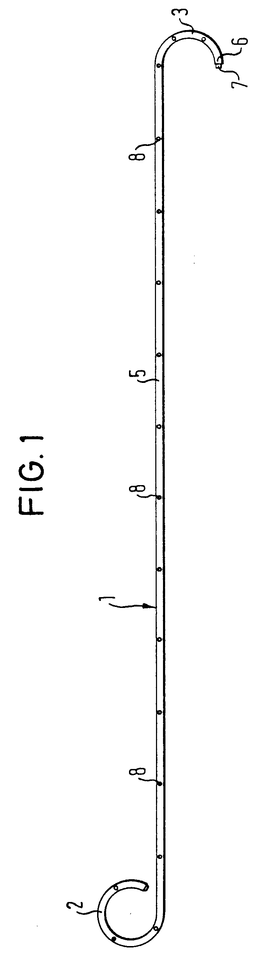

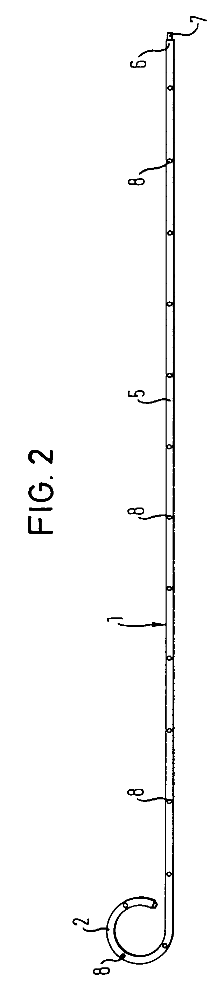

Das in Figur 2 dargestellte Ausführungsbeispiel entspricht im wesentlichen dem Ausführungsbeispiel nach Figur 1, wobei das distale Ende des Drainagerohrs 1 nicht als hakenförmige Umbiegung, sondern gerade verlaufend ausgebildet ist. Wie bereits beim Ausführungsbeispiel nach Figur 1 schließt sich an das distale Ende 6 des Hauptabschnitts 5 des Drainagerohrs 1 ein distaler Endabschnitt 7 des Drainagerohrs 1 an, der gegenüber dem Hauptabschnitt 5 einen verkleinerten Außendurchmesser besitzt.

Aus Figur 3 ist erkennbar, daß der distale Endabschnitt 7 des Drainagerohrs 1 durch ein separates Kopplungsrohr 10 gebildet wird, dessen Außendurchmesser im wesentlichen gleich dem Innendurchmesser des Hauptabschnitts 5 des Drainagerohrs 1 ist. Das Kopplungsrohr 10 ist innerhalb des Hauptabschnitts 5 des Drainagerohrs 1 angeordnet und über seinen gesamten Kontaktbereich 11 mit der Innenseite 12 des Hauptabschnitts 5 des Drainagerohrs 1 verklebt. Die Verklebungsstellen sind in Figur 3 durch die beiden dick ausgezogenen Linien 13 verdeutlicht.

Das distale Ende 6 des Hauptabschnitts 5 des Drainagerohrs 1 bildet eine im wesentlichen senkrecht zur Längsachse 4 verlaufende Stirnseite 14.

Auf den distalen Endabschnitt 7 des Drainagerohrs 1 ist ein Führungsrohr 15 aufgesteckt, dessen Außendurchmesser im wesentlichen gleich dem Außendurchmesser des Hauptabschnitts 5 des Drainagerohrs 1 ist. Der Innendurchmesser des Führungsrohrs 15 ist geringfügig kleiner als der Außendurchmesser des distalen Endabschnitts 7 des Drainagerohrs 1, so daß dieser im Preßsitz innerhalb des Führungsrohrs 15 angeordnet ist. Auf diese Weise können sowohl entlang der Längsachse 4 auf das Führungsrohr 15 einwirkende Kräfte als auch auf das Führungsrohr 15 einwirkende Rotationskräfte auf den distalen Endabschnitt 7 und den mit diesem fest verbundenen Hauptabschnitt 5 des Drainagerohrs 1 übertragen werden.

Während in Figur 3 zur Verdeutlichung zwischen der Stirnseite 14 des Hauptabschnitts 5 des Drainagerohrs 1 und der Stirnseite 16 des Führungsrohrs 15 ein Spalt 17 ausgebildet ist, wird bei der tatsächlichen Verwendung das Führungsrohr 15 bevorzugt so weit auf den distalen Endabschnitt 7 des Drainagerohrs 1 aufgeschoben, bis die beiden Stirnseiten 14 und 16 aneinander anliegen, so daß kein Spalt 17 vorhanden ist und die erfindungsgemäß ausgebildete Vorrichtung eine kontinuierliche verlaufende Außenfläche besitzt.

Innerhalb des Führungsrohrs 15 ist ein Schieberohr 18 angeordnet, dessen Außendurchmesser kleiner als der Innendurchmesser des Führungsrohrs 15 ist, so daß es leichtgängig innerhalb des Führungsrohrs 15 entlang der Längsachse 4 verschiebbar ist.

Der Außendurchmesser des Schieberohrs 18 ist gleichzeitig größer als der Innendurchmesser des distalen Endabschnitts 7 ausgebildet, so daß beim Verschieben des Schieberohrs 18 in Richtung des distalen Endabschnitts 7 des Drainagerohrs 1 die Stirnseite 19 des Schieberohrs 18 an der Stirnseite 20 des distalen Endabschnitts 7 zur Anlage kommt.

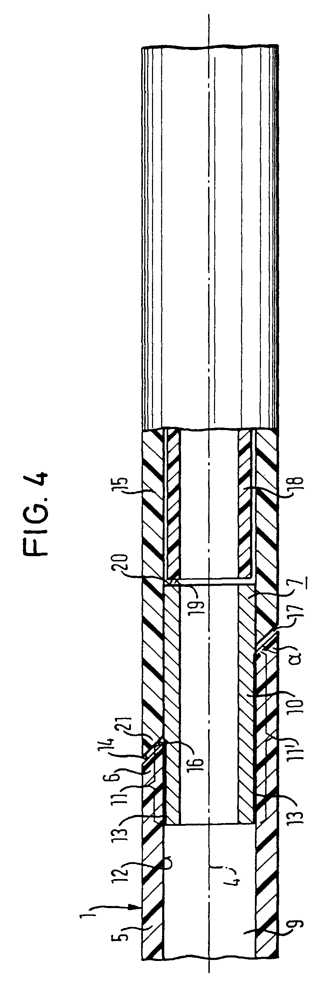

Bei dem in Figur 4 dargestellten Ausführungsbeispiel sind das distale Ende 6 des Hauptabschnitts 5 sowie das proximale Ende 21 des Führungsrohrs 15 abgeschrägt ausgebildet und schließen mit der Längsachse einen Winkel α von 45° ein. Der Kontaktbereich 11 zwischen der Außenseite des Kopplungsrohrs 10 und der Innenseite 12 des Hauptabschnitts 5 des Drainagerohrs 1 variiert zwischen der dargestellten Mindestweite 11 und der maximalen Weite 11'.

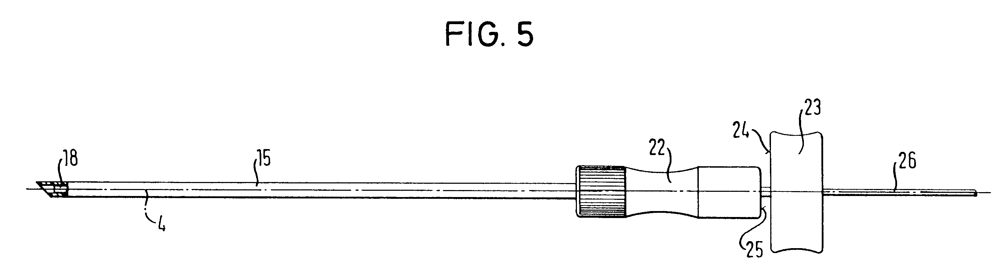

Aus Figur 5 ist zu erkennen, daß am distalen Ende des Führungsrohrs 15 ein Griff 22 vorgesehen ist, über den das Führungsrohr 15 zum einen in den Harnleiter eingeführt und zum anderen um die Längsachse 4 verdreht werden kann.

Weiterhin ist am distalen Ende des Schieberohrs 18 eine Handhabe 23 vorgesehen, über die das Schieberohr 18 in das Führungsrohr 15 eingeschoben werden kann. Darüber hinaus bildet die Handhabe 23 gleichzeitig eine Einschubbegrenzung für das Schieberohr 18, indem sie bei vollständigem Einschieben des Schieberohrs 18 in das Führungsrohr 15 mit ihrer Außenfläche 24 an der Stirnseite 25 des Griffs 22 zur Anlage kommt, wodurch eine weiteres Einschieben des Schieberohrs 18 verhindert wird.

Am distalen Ende der Handhabe 23 ist ein Führungsdraht 26 dargestellt, der sich durch das Schieberohr 18 und das Führungsrohr 15 bis zum Bereich des proximalen Endes des Drainagerohrs 1 erstreckt und durch den die flexiblen gekrümmten Enden 2, 3 des Drainagerohrs 1 während des Einführens in einer langgestreckten Stellung gehalten werden.

Im folgenden wird die Funktionsweise einer erfindungsgemäß ausgebildeten Vorrichtung näher beschrieben:

Das Führungsrohr 15 wird auf den distalen Endabschnitt 7 des Drainagerohrs 1 soweit aufgeschoben, bis die Stirnseite 16 des Führungsrohrs 15 an der Stirnseite 14 des Hauptabschnitts 5 des Drainagerohrs 1 zur Anlage kommt. Dadurch daß der Außendurchmesser des distalen Endabschnitts 7 geringfügig größer ist als der Innendurchmesser des Führungsrohrs 15, sind das Führungsrohrs 15 und der Endabschnitt 7 sowie der mit dem Endabschnitt 7 fest verbundene Hauptabschnitt 5 des Drainagerohrs 1 kraftschlüssig miteinander verbunden.

Anschließend wird der Führungsdraht 26 soweit in das Führungsrohr 15 eingeschoben, bis sein proximales Ende im Bereich des proximalen Endes des Drainagerohrs 1 angeordnet ist, so daß die flexiblen gekrümmten Enden 2, 3 des Drainagerohrs 1 in eine langgestreckte Form gebracht werden.

Das Drainagerohr 1 wird in den Harnleiter des Patienten eingeführt, wobei ein Verschieben des Drainagerohrs 1 auch nach vollständigem Einführen in den Harnleiter durch fortwährendes Nachschieben des Führungsrohrs 15 mittels des Griffs 22 erfolgt. Eventuelle Probleme beim Einführen aufgrund von Biegungen des Harnleiters können durch Drehen am Griff 22 gelöst werden, da sich die auf den Griff 22 ausgeübte Drehbewegung auf das Führungsrohr 15 und das mit diesem kraftschlüssig verbundene Drainagerohr 1 überträgt.

Das Drainagerohr 1 wird soweit in den Harnleiter eingeführt, bis das proximale Ende des Drainagerohrs 1 in der Niere und das distale Ende des Drainagerohrs 1 in der Blase angeordnet sind.

In dieser Position wird das Schieberohr 18 in das außerhalb des Körpers des Patienten gelegene distale Ende des Führungsrohrs 15 eingesetzt und soweit in das Führungsrohr 15 eingeschoben, bis die Stirnseite 19 des Schieberohrs 18 an der Stirnseite 20 des distalen Endabschnitts 7 des Drainagerohrs 1 zur Anlage kommt. Da das Schieberohr 18 ausschließlich innerhalb des geschlossenen Führungsrohrs 15 in den Harnleiter des Patienten eingeführt wird, ist eine Beeinträchtigung der Innenwand des Harnleiters nicht möglich.

Durch Halten bzw. leichtes weiteres Vorwärtsschieben des Schieberohrs 18 und gleichzeitiges Zurückziehen des Führungsrohrs 15 wird dieses von dem distalen Endabschnitt 7 abgezogen, da der Endabschnitt 7 des Drainagerohrs 1 von dem Schieberohr 18 in seiner Stellung gehalten wird.

Nachdem das Führungsrohr 15 vollständig von dem distalen Endabschnitt 7 des Drainagerohrs 1 abgezogen ist, kann das Führungsrohr 15 zusammen mit dem Schieberohr 18 aus dem Harnleiter des Patienten herausgezogen werden, so daß das Drainagerohr 1 im Harnleiter zwischen der Niere und der Blase positioniert bleibt.

Bei dem Ausführungsbeispiel gemäß Figur 4 wird durch die abgeschrägten Stirnseiten 14 und 16 des Hauptabschnitts 5 bzw. des Führungsrohrs 15 eine zwischen dem Drainagerohr 1 und dem Führungsrohr 15 zusätzlich wirkende, form- und kraftschlüssige Verbindung erreicht, durch die eine Drehbewegung des Führungsrohrs 15 sicher auf das Drainagerohr 1 übertragen wird. Somit ist sowohl die Verschiebung des Drainagerohrs 1 in gebogenen Abschnitten des Harnleiters gewährleistet als auch eine Verdrehung des Drainagerohrs 1 an seiner Endposition zwischen Niere und Blase erreichbar, durch die eine optimale Plazierung der gebogenen Enden 2, 3 des Drainagerohrs 1 innerhalb der Niere bzw. der Blase erreicht werden kann.

## Patentansprüche

1. Vorrichtung zur Drainage an einem Patienten mit einem Drainagerohr (1), einem mit dem Drainagerohr (1) koppelbaren Führungsrohr (15) und einem Schiebeelement (18),
dadurch **gekennzeichnet,**
daß zumindest ein distaler Endabschnitt (7) des Drainagerohrs (1) innerhalb des Führungsrohrs (15) angeordnet ist,
und daß das Schiebeelement (18) innerhalb des Führungsrohrs (15) zum Entkoppeln von Drainagerohr (1) und Führungsrohr (15) gegen den distalen Endabschnitt (7) des Drainagerohrs (1) verschiebbar ist.

2. Vorrichtung nach Anspruch 1,
dadurch **gekennzeichnet,**
daß das Drainagerohr (1) und das Führungsrohr (15) über den distalen Endabschnitt (7) des Drainagerohrs (1) miteinander insbesondere kraftschlüssig verkoppelbar sind und/oder daß der Außendurchmesser des distalen Endabschnitts (7) des Drainagerohrs (1) im wesentlichen gleich dem Innendurchmesser des Führungsrohrs (15) ist.

3. Vorrichtung nach einem der Ansprüche 1 oder 2,
dadurch **gekennzeichnet,**
daß der distale Endabschnitt (7) des Drainagerohrs (1) im wesentlichen im Preßsitz innerhalb des Führungsrohrs (15) angeordnet ist, so daß durch die zwischen der Außenseite des distalen Endabschnitts (7) und der Innenseite des Führungsrohrs (15) wirkende Reibungskraft eine im wesentlichen kraftschlüssige Kopplung gebildet ist.

4. Vorrichtung nach einem der vorhergehenden Ansprüche,
dadurch **gekennzeichnet,**
daß der distale Endabschnitt (7) des Drainagerohrs (1) als insbesondere röhrenförmiges, separates Element (10) ausgebildet ist, das mit einem Hauptabschnitt (5) des Drainagerohrs (1) verbunden, insbesondere verklebt ist.

5. Vorrichtung nach einem der Ansprüche 1 bis 3,
dadurch **gekennzeichnet,**
daß der distale Endabschnitt (7) des Drainagerohrs (1) einstückig mit einem Hauptabschnitt (5) des Drainagerohrs (1) ausgebildet, insbesondere an diesen angeformt ist.

6. Vorrichtung nach Anspruch 4 oder 5,
dadurch **gekennzeichnet,**
daß der Hauptabschnitt (5) des Drainagerohrs (1) und das Führungsrohr (15) im wesentlichen denselben Außendurchmesser besitzen und/oder daß das Drainagerohr (1) und das Führungsrohr (15) über am distalen Ende (6) des Hauptabschnitts (5) des Drainagerohrs (1) und am proximalen Ende (21) des Führungsrohrs vorgesehene Kopplungselemente miteinander koppelbar sind.

7. Vorrichtung nach einem der Ansprüche 4 bis 6,
dadurch **gekennzeichnet,**
daß das distale Ende (6) des Hauptabschnitts (5) des Drainagerohrs (1) und das proximale Ende (21) des Führungsrohrs (15) insbesondere im wesentlichen komplementär zueinander abgeschrägt ausgebildet sind, wobei insbesondere die abgeschrägten Enden (6, 21) mit der Längsachse (4) des Drainagerohrs (1) bzw. des Führungsrohrs (15) einen Winkel (α) zwischen 90° und 30°, insbesondere zwischen 90° und 45° einschließen.

8. Vorrichtung nach einem der Ansprüche 6 oder 7,
dadurch **gekennzeichnet,**
daß das am Drainagerohr (1) vorgesehene Kopplungselement bzw. eines der am Drainagerohr (1) vorgesehenen Kopplungselemente als insbesondere V-förmige Ausnehmung und das am Führungsrohr (15) vorgesehene Kopplungselement bzw. eines der am Führungsrohr (15) vorgesehenen Kopplungselemente als insbesondere im wesentlichen komplementär dazu ausgebildeter Vorsprung ausgebildet sind.

9. Vorrichtung nach einem der Ansprüche 6 bis 8,
dadurch **gekennzeichnet,**
daß das am Führungsrohr (15) vorgesehene Kopplungselement bzw. eines der am Führungsrohr (15) vorgesehenen Kopplungselemente als insbesondere V-förmige Ausnehmung und das am Drainagerohr (1) vorgesehene Kopplungselement bzw. eines der am Drainagerohr (1) vorgesehenen Kopplungselemente als insbesondere im wesentlichen komplementär dazu ausgebildeter Vorsprung ausgebildet sind.

10. Vorrichtung nach einem der vorhergehenden Ansprüche,
dadurch **gekennzeichnet,**
daß das Schiebeelement als Schieberohr (18) ausgebildet ist und daß insbesondere dessen Außendurchmesser im wesentlichen gleich oder geringfügig kleiner als der Innendurchmesser des Führungsrohrs (15) ist und/oder daß das Drainagerohr (1) und/oder das Führungsrohr (15) und/oder das Schiebeelement (18) aus körperverträglichem Kunststoff hergestellt sind.
